# EUROPÄISCHE PATENTSCHRIFT

(11) **EP 0 682 911 B1**
(45) Veröffentlichungstag und Bekanntmachung des Hinweises auf die Patenterteilung: **08.03.2000**
(21) Anmeldenummer: 95250118.7
(22) Anmeldetag: 18.05.1995
(51) Int. Cl.: A61B 5/042

(54) **Endoskopisch einsetzbare Elektrodenanordnung**
Electrode array applied by endoscope
Formation d'électrodes à appliquer par endoscope

(30) Priorität: 19.05.1994 DE 4418022
(43) Veröffentlichungstag der Anmeldung: 22.11.1995
(73) Patentinhaber: BIOTRONIK Mess- und Therapiegeräte GmbH & Co Ingenieurbüro Berlin, D-12359 Berlin (DE)
(72) Erfinder: Akchurin, Renat S., Prof., 121552 Moskau (RU)
(74) Vertreter: Eisenführ, Speiser & Partner

(56) Entgegenhaltungen:
- EP-A- 0 095 726
- EP-A- 0 099 077
- DE-C- 3 718 139
- DE-C- 4 205 336
- US-A- 4 628 937
- PROCEEDINGS OF THE EIGHTH ANNUAL CONFERENCE OF THE IEEE/EMB SOCIETY, 10. November 1986, FORT WORTH (US) Seiten 1292 - 1295 I.D. PARSON ET AL. 'Analog Cardiac Mapping'
- BIOMEDIZINISCHE TECHNIK, Bd.24, Nr.11, November 1979, BERLIN (DE) Seiten 267 - 270 J.M.T. DE BAKKER ET AL. 'Methode zur Erfassung multipler epikardialer Signale'
- MüNCHENER MEDIZINISCHE WOCHENSCHRIFT, Bd.115, Nr.24, 1973, MüNCHEN (DE) Seiten 1141 - 1144 G. SCHMITT ET AL. 'Klinische Erfahrungen mit einer neuentwickelten Katheter-Elektrode'

## Beschreibung

Die Erfindung betrifft eine Elektrodenanordnung der im Oberbegriff des Anspruchs 1 angegebenen Art.

Aus der Pränataldiagnostik sind Meßsonden mit aus dem Stirnwandbereich eines Katheters ausfahr- und -spreizbaren Elektroden bekannt, die im geschlossenen Zustand intravaginal eingeführt und aus denen nach der Einführung zwei Elektroden ausgefahren werden, die in die fetale Kopfhaut eindringen und zur Erfassung des fetalen EKG während des Geburtsvorganges dienen. Derartige Meßsonden sind etwa in DE 33 37 188 A1 und EP 0 007 702 B1 beschrieben. Sie eignen sich vom mechanischen Aufbau her nicht für Messungen direkt am Herzen und grundsätzlich nicht für kartierende ("Mapping"-)Untersuchungen.

Solche kartierenden Untersuchungen, deren Ziel in der Bestimmung einer räumlichen Potentialverteilung besteht, haben in den letzten Jahren im Zusammenhang mit der Diagnose und Therapie tachykarder Herzrhythmusstörungen Bedeutung gewonnen. Dabei wird die Ausbreitung der elektrischen Erregung im Herzen kartiert, um anhand der Abweichungen gegenüber einem gesunden Herzen auf Defekte der Leitungswege schließen zu können. Im Anschluß an die Lokalisierung von Defekten bzw. pathogenen Leitungsstrukturen kann an den entsprechenden Stellen der Herzwandungen mitttels eines hochfrequenten Stromstoßes eine Ablation, d.h. eine Zerstörung von Gewebsabschnitten, vorgenommen werden, um die wege der pathologischen Erregungsleitung zu unterbrechen und damit die Tachykardie zu therapieren.

Ein einfacher, endokardial einzusetzender Elektrodenkatheter für das Tachykardie-Mapping ist aus DE 40 25 369 A1 bekannt. Dieser Katheter eignet sich lediglich für grob orientierende Untersuchungen.

Dies gilt auch für den aus DE 35 29 063 bekannten - ebenfalls endokardial einzusetzenden - Katheter, dessen Anwendung die Einhaltung relativ großer Abstände der auf der Außenseite eines Ballons angeordneten Mehrzahl von Elektroden zur Herzwandung erfordert und mit dem daher die Potentialverteilung innerhalb derer prinzipiell nur mit großer Ungenauigkeit bestimmt werden kann.

Aus US 5,237,996 ist ein endokardial einzusetzender Mapping-Katheter bekannt, bei dem nach Einführung in den Ventrikel aus dem Vorderteil eines Katheterkopfes mehrere über dessen Umfang verteilt angeordnete feine, federnde Elektrodendrähte schräg nach vorn ausgefahren werden können. Diese Elektrodendrähte kommen im ausgefahrenen Zustand mit der Herzwandung in Berührung und erlauben dort eine relativ genaue Potentialerfassung. Da alle Elektroden jedoch im wesentlichen in einer vertikalen Ebene die Herzwandung berühren, sind für eine Kartierung mehrere Meßschritte unter sukzessivem Vorschieben des Katheters in den Ventrikel nötig.

In US 5,239,999 sind demgegenüber weiter verbesserte, allerdings konstruktiv aufwendige, Ausführungen von endokardial einzusetzenden Mapping-Kathetern beschrieben, bei denen eine große Anzahl von Elektroden auf zur Nachbildung eines ellipsoidalen Umrisses ausspreizbaren Elementen vorgesehen ist.

Die genannten Anordnungen sind - neben ihren sonstigen Nachteilen - aufgrund ihrer geometrischen Gestalt nicht für das epikardiale Mapping geeignet.

Epikardial einzusetzende Elektroden sind aus EP 0 095 726 bekannt. Die dort beschriebenen, zum Anlegen von Defibrillationsimpulsen vorgesehenen, Elektroden haben eine paddelartige Gestalt und werden mittels einer gesonderten Kanüle in den perikardialen Raum eingeführt. Mit mehreren solchen Elektroden sind bestimmte räumliche Feldverteilungen während eines Kardioversionsversuches realisierbar.

Für eine Kartierung der Herzaktionspotentiale sind jedoch einzelne derartige Elektroden überhaupt nicht und eine Konfiguration aus mehreren Elektroden - unter anderem wegen des hohen Aufwandes beim Anlegen - nur sehr bedingt brauchbar.

Aus I.D. Parson et al "Analog Cardiac Mapping", Eighth Annual Conference of the IEEE/EMB Society (10. November 1986), 1292, ist eine Kombination aus einer endokardialen und einer epikardialen Flächenelektrode für die kardiale Kartierung bekannt, bei der die Implantation des epikardialen Teils eine offene Herzoperation erfordert. Dies gilt für eine von J.M.T. DeBakker et al: "Methode zur Erfassung multipler epikardialer Signale", Biomedizinische Technik 24 (1979), 267, beschriebene netzartige epikardiale Kartierungselekrodenanordnung.

Der Erfindung liegt die Aufgabe zugrunde, eine Elektrodenanordnung der eingangs genannten Gattung anzugeben, die für die - insbesondere epikardiale - Tachykardiekartierung geeignet und einfach herstell- und anwendbar ist.

Diese Aufgabe wird durch eine Elektrodenanordnung mit den Merkmalen des Anspruchs 1 gelöst.

Die Erfindung schließt den Gedanken ein, einen von der Geometrie und Betätigung her für die gleichzeitige endoskopische Einführung einer Mehrzahl von Elektroden in den peridardialen Raum und eine von dort aus erfolgende Umfassung der Herzspitze und unteren Bereiche des Epikards mit den Elektroden geeigneten Elektroden-Katheter zu schaffen.

Für diesen Zweck eignet sich eine Realisierung der Elektroden auf einer auf einfache Weise aus dem Katheterkopf bzw. -körper aufklapp- oder auffaltbaren Elektrodenträgerfläche, die in Arbeitsstellung V- bzw. Trichter- oder U-bzw. Schüsselgestalt annimmt. Die Anwendung einer solchen Anordnung ist jedoch nicht auf diesen Einsatz beschränkt, sondern kann - bei Ausführung in geeigneten Abmessungen - auch endokardial oder ggfs. bei einer elektrografischen Untersuchung anderer Körperteile erfolgen.

Eine besonders einfach herstell- wie bedienbare und robuste Ausführung ist derart, daß der Elektrodenträger zwei langgestreckte, gegeneinander verschwenkbare Schenkel aus steifem oder halbsteifem Material aufweist, deren Längsachsen zum Einführen in den Körper im wesentlichen parallel zur Katheterlängsachse liegen und im ausgeklappten bzw. aufgefalteten Zustand mit dieser eine spitzen Winkel einschließen. Es können auch mehr als zwei Schenkel vorgesehen sein.

Weiterhin kann - alternativ zur vorgenannten Ausführung oder in Kombination mit drei oder mehr der genannten Schenkel - der Elektrodenträger eine gitter- bzw. netzartige Struktur oder auch eine vollflächige Lage aus flexiblem Material mit halbsteifen. elastischen Aufspreizungselementen (etwa in Art eines Schirmes) aufweisen. Die Schenkel können die Aufspreizungselemente bilden, es sind aber aber auch Spiralfedern aus Metall oder Kunststoff o.ä. einsetzbar.

Auf diesem Elektrodenträger kann eine Mehrzahl einzelner, voneinander isolierter Elektroden angeordnet sein, wobei die Anzahl - je nach konkreter Meßaufgabe - vorzugsweise zwischen 10 und 200 liegt. Eine sehr genaue Potentialkartierung läßt sich mit einer Anordnung erreichen, bei der der Abstand der einzelnen Elektroden voneinander etwa 1 cm beträgt. Die Elektroden bestehen aus einem hoch leitfähigen biokompatiblen Material, vorzugsweise TiN oder IrO₂.

Übersichtliche geometrische Verhältnisse und damit Möglichkeiten einer vereinfachten und dennoch präzisen Auswertung ergeben sich, wenn die Elektroden im wesentlichen in Zeilen und Spalten ausgerichtet, d.h. matrixartig, angeordnet sind.

Diese Anordnung bietet zudem die vorteilhafte Möglichkeit einer vereinfachten Beschaltung mit einer bei großer Elektrodenzahl wesentlich verringerten Leitungszahl, indem jede Spalte und Zeile der matrixartigen Anordnung mit einer separaten Zuleitung zum Abgreifen (oder ggfs. auch Zuführen) einer elektrischen Spannung verbunden wird. Aktiviert ist dann jeweils die an einem Kreuzungspunkt einer aktivbierten Zeilen- und einer aktivierten Spalten-Leitung angeordnete Elektrode.

Konstruktiv besonders einfach ist eine Ausführung, bei der das flexible Material eine Widerstandsfolie aufweist und an seiner (im ebenen Zustand etwa drei- oder rechteckigen) Umfangslinie eine Mehrzahl (bei einem Dreieck drei, bei einem Rechteck vier usw.) von Anschlüssen vorgesehen ist, von denen jeder mit einer separaten Zuleitung verbunden ist. Der Ort des Auftretens einer Potentialspitze kann hierbei prinzipiell aus den Quotienten der an den einzelnen Anschlüssen abgegriffenen Spannungen bestimmt werden, was allerdings eine leistungsfähige Auswertungseinheit voraussetzt.

In der Ausführung zur epikardialen Tachykardiekartierung beträgt die Länge des Katheterkopfes zwischen 10 und 15 cm, vorzugsweise 12 cm, sein Durchmesser zwischen 1 und 3, vorzugsweise 2 cm, und die Öffnungsweite des V- oder U-förmig aufgeklappten bzw. aufgefalteten Elektrodenträgers 10 bis 15 cm, vorzugsweise etwa 12 cm.

Der Katheterkopf und/oder der Elektrodenträger können in vorteilhafter Ausführung ein PTFE-, Polyamid- oder Polyethylensubstrat aufweisen.

Eine für bestimmte Anwendungen vorteilhafte Kombination von elektrografischer und optischer Unterscuhung oder ggfs. eine Ablation unter optischer Kontrolle wird mit einer Ausbildung möglich, bei der im Katheterkopf eine optische bzw. optoelektronische Anordnung zur visuellen oder videotechnischen Beobachtung des Untersuchungsortes vorgesehen ist und die Hülse eine entsprechende, mit der optischen Anordnung verbundene Signalleitung aufweist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Es zeigen:
Figur 1a eine schematische Darstellung einer Gesamtanordnung zur epikardialen Tachykardiekartierung mit einer Elektrodenanordnung nach einer Ausführungsform der Erfindung,
Figur 1b eine schematische Darstellung des in Fig. 1a gezeigten Elektrodenkatheters im geschlossenen Zustand,
Figur 2a eine schematische Darstellung einer im perikardialen Raum plazierten Elektrodenanordnung nach einer weiteren Ausführungsform der Erfindung und
Figur 2b eine schematische Darstellung des in Fig. 2a gezeigten Elektrodenkatheters im geschlossenen Zustand.

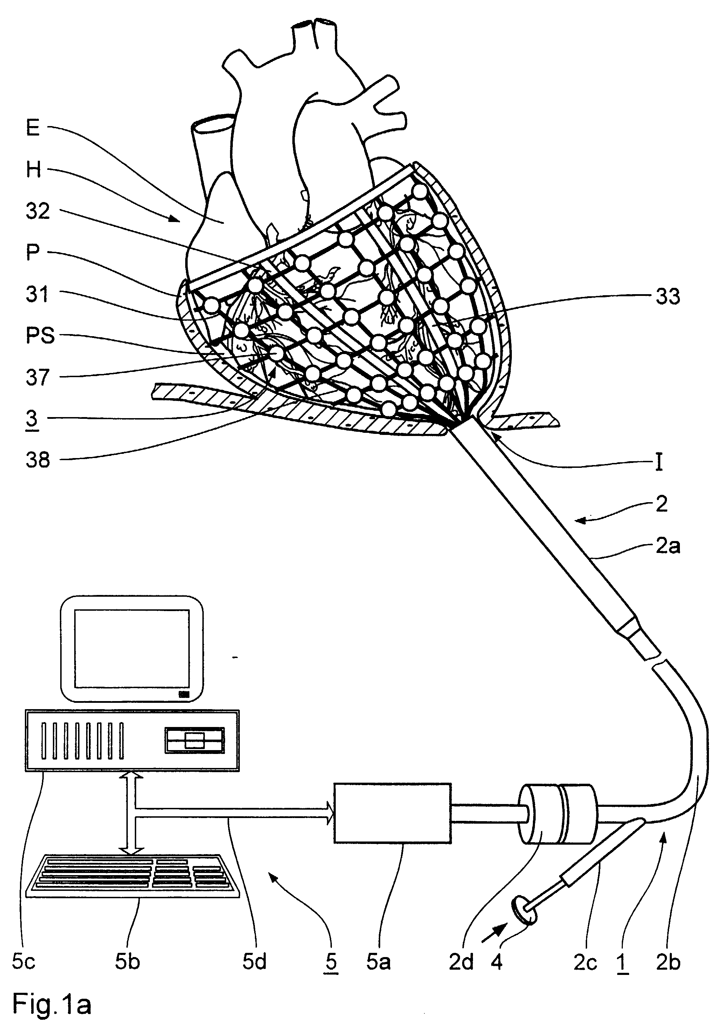

Fig. 1a zeigt in schematischer, teilweise frontal geschnittener Darstellung eine Untersuchungsanordnung 1 zum Tachykardie-Mapping an einem Herzen H. Die Untersuchungsanordnung schließt eine am distalen Ende eines Katheters 2 angeordnete, im perikardialen Raum PS zwischen dem Herzen H und dem - in Frontalebenen geschnitten dargestellten - Herzbeutel (perikard) P plazierte, den unteren Bereich der Herzwandung (des Epikard) E korbartig umgreifende Elektrodenanordnung 3 ein. Die Elektrodenanordnung 3 erstreckt sich aus dem distalen Ende eines Katheterkopfes 2a heraus, das durch eine Inzision I im Herzbeutel P in den perikardialen Raum PS eingeführt ist.

Der Katheterkopf 2a ist mit einer flexiblen Katheterleitung 2b verbunden, die (was in Fig. 1a nicht gezeigt ist) Elektrodenzuleitungen und ein Kraftübertragungselement für die Betätigung der Anordnung nach außerhalb des Körpers führt und am proximalen Ende einen Abzweigungsabschnitt 2c mit einem Griffstück 4 zur Betätigung der Elektrodenanordnung und eine Steckverbindung 2d aufweist.

Über die Steckverbindung 2d ist die Elektrodenanordnung mit einer Schnittstelle 5a eines Steuer- und Auswertungssystems 5 verbunden, als dessen weitere Hauptkomponenten symbolisch eine Steuer- und Stromversorgungseinheit 5b, ein Auswertungsrechner 5c und ein Bussystem 5d dargestellt sind.

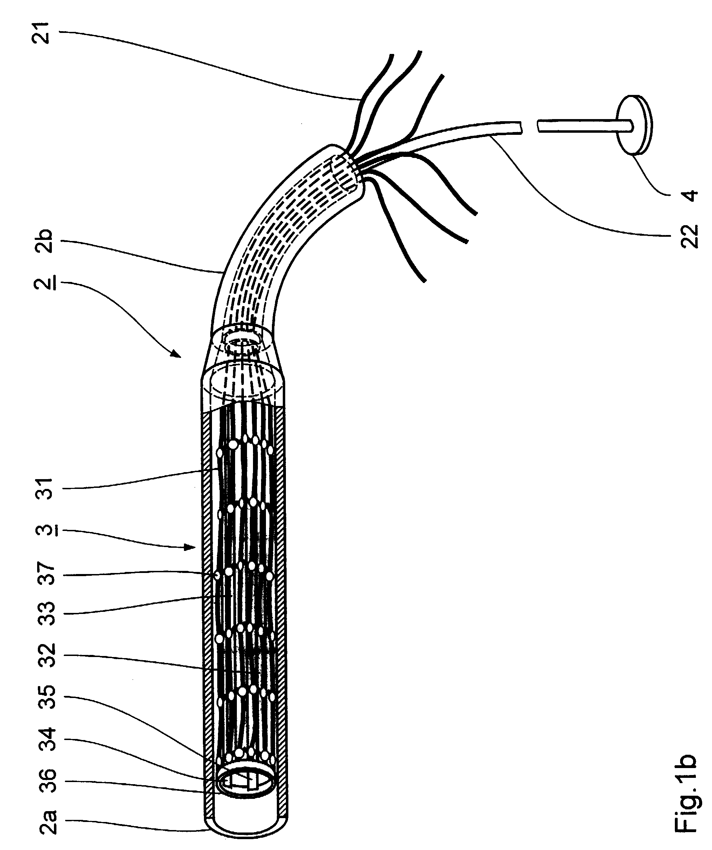

Die Elektrodenanordnung 3 besteht aus einem der Form des Herzens angepaßt korb- bzw. schüsselförmigen, grobmaschigen Netz 31 mit einer größten Maschenweite von etwa 1 cm. Das Netz weist vier flexible, in annähernd axialer Richtung aus dem Katheterkopf 2a austretende PTFE-ummantelte Edelstahl-Versteifungsdrähte 32 bis 35 (von denen die Stäbe 34 und 35 nur in Fig. 1b zu erkennen sind) von ca. 12 cm Länge auf, deren distale Enden über einen Teleskopfederring 36, der das Netz 31 distal begrenzt und seinen Rand versteift, miteinander verbunden sind und auf Abstand gehalten werden. Die Versteifungsstäbe 32 bis 35 und der Federring 36 und damit auch das Netz 31 liegen an der inneren Oberfläche des Perikards und im wesentlichen auch dicht an der äußeren Oberfläche des Epikards an.

An den Knotenpunkten des Netzes sind jeweils kreisscheibenförmige Titannitrid-Elektroden 37 von etwa 1-2 mm Durchmesser angeordnet, so daß das Epikard von einer matrixartigen Elektrodenanordnung umgeben ist. Die Elektroden 37 mit der Steuer- und Auswertungseinheit 5 verbindende Elektrodenleitungen verlaufen in den Längsfäden 38 des Netzes zur Strinseite des Katheterkopfes und weiter in dessen Innerem, wie in Fig. 1b genauer zu sehen ist.

Fig. 1b zeigt den vorderen Teil des Katheters 2 mit dem Elektroden-Netz 3 aus Fig. 1a im zusammengefalteten Zustand, wie er bei der Einführung des Katheters in den perikardialen Raum PS vorliegt. (Die Ausbildung des proximalen Endes des Katheters und der Steuer- und Auswertungseinrichtung ist Fig. 1a zu entnehmen.)

Wie in Fig. 1b deutlich zu erkennen ist, ist die Elektrodenanordnung 3 vor und bei der Einführung des Katheters in dem als steifer Hohlzylinder mit etwa 12 cm Länge und 2 cm Durchmesser - etwa aus PTFE - ausgeführten Katheterkopf 2a derart zusammengefaltet, daß die Versteifungsdrähte 32 bis 35 in etwa parallel zur Katheterlängsachse liegen und der Teleskopfederring 36 soweit zusammengeschoben ist, daß sein Umfang dem inneren Umfang des Katheterkopfes 2a entspricht.

In der Figur ist auch verdeutlicht, daß die Katheterleitung 2b Elektrodenzuleitungen 21 (von denen beispielhaft nur sechs gezeigt sind) und einen die Versteifungsdrähte 32 bis 35 mit dem Griffstück 4 verbindenden Draht 22 aufnimmt, über den das Herausschieben der Elektrodenanordnung 3 aus dem Katheterkopf 2a und ihr Aufspreizen um das Epikard herum bewirkt wird, nachdem der Katheter 2 korrekt plaziert wurde. Der Draht 22 kann direkt aus miteinander verdrillten oder anderweitig verbundenen proximalen Verlängerungen der Versteifungsdrähte 32 bis 35 gebildet oder ein gesondertes, mit den proximalen Enden der Versteifungsdrähte fest verbundenes (etwa verschweißtes oder hartverlötetes), Element sein.

Das epikardiale Anlegen der Elektrodenanordnung kann etwa derart erfolgen, daß zunächst eine transkutane und transperikardiale, von dorsal nach ventral und schräg nach oben gerichtete Inzision im Bereich der Herzspitze vorgenommen und durch diese das distale Ende des Katheterkopfes 2a schräg von unten zur Herzspitze vorgeschoben wird. Danach wird durch Vorschieben des Griffstücks 4 in distaler Richtung das Herausschieben der Versteifungsdrähte 32 bis 35 und mit diesen auch des Federringes 36 und des Netzes 31 aus dem katheterkopf bewerkstelligt, wozu insbesondere Kontraktionsphasen des Herzens genutzt werden.

Wenn die Elektrodenanordnung vollständig ausgefaltet ist und das Epikard umschließt, wird die Steckverbindung 2d geschlossen und ein - als solches bekanntes - Steuerprogramm zur Tachykardiekartierung gestartet, in dessen Verlauf durch geeignete Stimulationsimpulsfolgen, die über die beschriebene oder eine gesonderte, intrakardiale Elektroden anordnung appliziert werden, künstlich ein temporärer Tachykardiezustand des zu untersuchenden Herzens hervorgerufen wird. Während dieser Zustand herrscht, werden über jede der Elektroden 37 jeweils Spannungssignale und somit über die Elektrodenanordnung insgesamt - bei entsprechender Zuordnung und Auswertung der Spannungssignale mittels der Auswertungseinrichtung 5c - ein Bild der Potentialverteilung im Epikard aufgenommen, das Aufschluß über Erregungszentren und Wege der Erregungsleitung bei der tachykarden Arrhythmie gibt.

Dieses Abbild der lokalen Verteilung der Herzaktionspotentiale beim konkreten Patienten kann die Grundlage für eine gezielte Elektrotherapie spontan auftretender Tachykardien oder für eine operative Zerstörung von für die Entstehung der Tachykardie relevanten Leitungswegen, etwa auf dem Wege der Ablation durch HF-Impulse, sein.

Die beschriebene Elektrodenanordnung kann dabei auch für die Therapie genutzt werden, indem über die Steuereinheit 5b HF-Impulse erzeugt und den entsprechend dem Kartierungsergebnis vorbestimmten Elektroden zugeführt werden. Es ist grundsätzlich auch möglich, eine auf die beschriebene Weise einmal in den perikardialen Raum eingebrachte, entsprechend modifizierte Elektrodenanordnung dort zu belassen und zur Applikation von Defibrillationsimpulsen im Bedarfsfall zu nutzen. Dies erfordert insbesondere das Vorsehen einer lösbaren Verbindung zwischen dem Elektroden-Netz und dem Katheter (das wieder entfernt werden muß) und von geeigneten Leitungsverbindungen zu einem Standby-Defibrillator. Zweckmäßig ist auch eine Ausbildung derart, daß die Versteifungsstäbe nach dem Einbringen des Netzes mit dem Katheter wieder entfernt werden können.

In einer modifizierten Anordnung können die Elektroden 37 in einer der oben beschriebenen ansonsten entsprechenden Anordnung jeweils mit einer von m durchgehenden Zeilen- und n Spalten-Leitungen einer mxn-Ansteuerungsmatrix verbunden sein. In diesem Fall werden für eine Messung sukzessive in an sich bekannter Weise die Zeilen- und Spaltenleitungen abgetastet bzw. empfindlich geschaltet derart, daß beispielsweise die Elektrode auf dem Maschenknoten (3,4) über eine UND-Verschaltung der Zeilenleitung 3 und der Spaltenleitung 4 in der Auswertungseinheit 5c abgefragt wird. Durch diese Abwandlung ist bei großen Elektrodenzahlen eine drastische Verringerung der Anzahl der Elektrodenzuleitungen möglich: Während für m x n Elektroden auch m x n einzelne Zuleitungen erforderlich sind, reduziert sich die Anzahl der Zuleitungen bei Verschaltung in einer mxn-Matrix auf m + n.

Die vorbeschriebene Anordnung kann weiterhin dergestalt modifiziert sein, daß anstelle des Netzes 31 eine Widerstandsfolie mit zueinander definiert positionierten Abgriffen vorgesehen ist, die nach dem Einführen und Auffalten der modifizierten Anordnung das Herz umspannt. In einer Widerstandsfolie, die als solche - allerdings mit taktiler Betätigung - etwa aus interaktiven Displays bekannt ist, können durch Herzaktionspotentiale zu den Abgriffspunkten hin gerichtete Stromflüsse induziert werden. Die Bestimmung der Spannungsabfälle an den einzelnen Abgriffspunkten der als kontinuierliches Widerstandsnetzwerk zu beschreibenden Folie ermöglicht grundsätzlich die Lokalisierung der Herzaktionspotentiale und damit ebenso ein Tachykardie-Mapping wie die oben beschriebene Anordnung mit einzelnen Abtastelektroden 37.

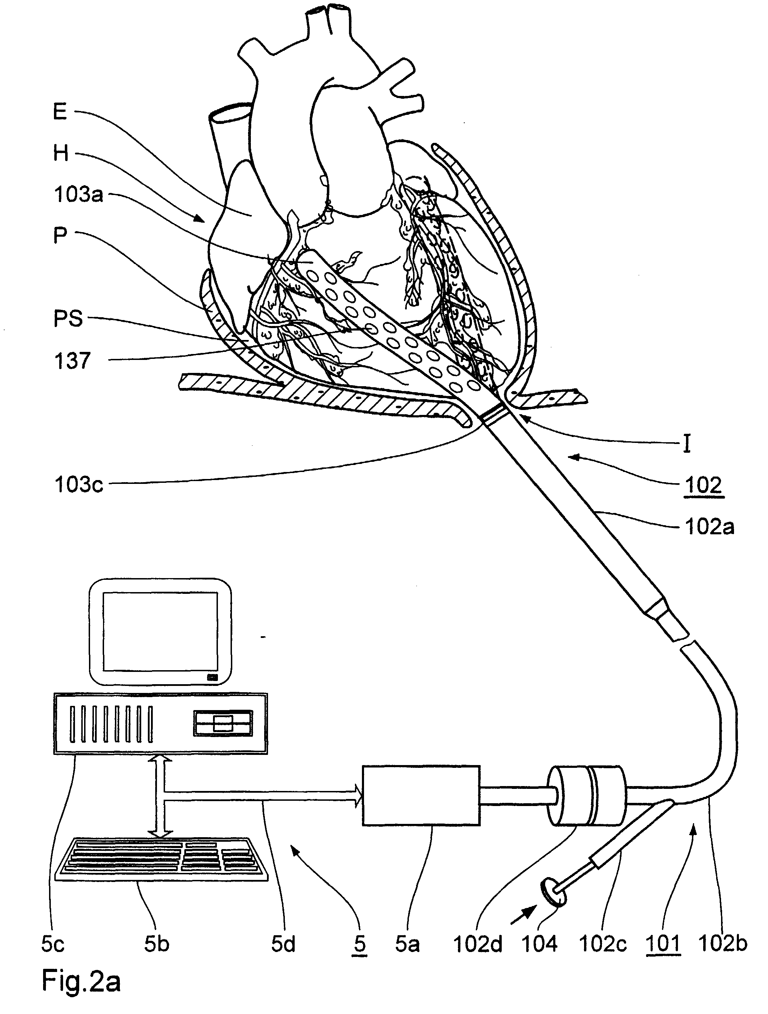

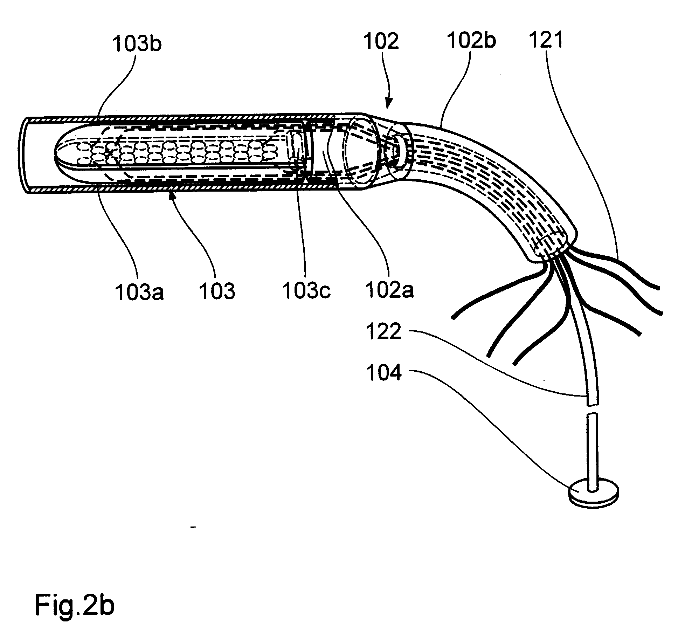

Fig. 2a zeigt in schematischer Darstellung einer weiteren Ausführungsform eine ebenfalls im perikardialen Raum eines Herzens H eingesetzte Anordnung 101, und Fig. 2b zeigt die Anordnung im geschlossenen Zustand vor der Einführung. Die Darstellung des herzens und seiner Umgebung sowie des Steuer- und Auswertungssystems - einschließlich der Bezugszeichen - entspricht Fig. 1a.

Hier sind am distalen Ende eines Katheters 102 zwei schnabelartig klappbare, jeweils im wesentlichen halbzylindrisch geformte Elektrodenträger 103a und 103b angebracht, die jeweils eine Mehrzahl von einzelnen Elektroden 137 tragen. Die Elektrodenträger 103a und 103b sind über eine Achse 103c gegeneinander verschwenkbar und über einen steifen Katheterabschnitt 102a mit einer flexiblen Katheterleitung 102b verbunden, die (was in Fig. 2a nicht gezeigt ist) Elektrodenzuleitungen und ein Kraftübertragungselement für die Betätigung der Anordnung nach außerhalb des Körpers führt. Analog zur Anordnung nach Fig. 1a sind am proximalen Ende ein Abzweigungsabschnitt 102c mit einem Griffstück 104 zur Betätigung der Elektrodenträger und eine Steckverbindung 102d angeordnet.

Die Elektrodenträger 103a und 103b umgreifen in Arbeitsstellung von unten einen Abschnitt des Herzens, wobei jeweils einer an der Herzwandung ventral und der andere dorsal an ihr anliegt, so daß die Elektroden das reizbare Herzgewebe kontaktieren und auf ähnliche Weise wie oben beschrieben einerseits Herzaktionspotentiale abtasten oder andererseits dem Herzen Reizimpulse zuführen können.

In Fig. 2b ist zu erkennen, daß die Elektrodenträger 103a und 103b im zusammengeklappten Zustand einen den steifen Katheterabschnitt 102a distal fortsetzenden, annähernd zylindrischen Katheterkopf 103 bilden, der auf ähnliche Weise wie die Anordnung nach Fig. 1 in den Körper des Patienten eingeführt wird. In der letzten Phase der Einführung in den perikardialen Raum werden über das Griffstück 104 und den Draht 122 die Schenkel bzw. Elektrodenträger 103a und 103b unter weiterem Vorschieben sukzessive auseinandergeklappt, um den Epikard dazwischen zu erfassen.

In der Figur ist auch verdeutlicht, daß die Katheterleitung 102b Elektrodenzuleitungen 121 (von denen beispielhaft nur sechs gezeigt sind) und einen die proximalen Enden der Schenkel 103a und 103b mit dem Griffstück 104 verbindenden Draht 122 aufnimmt. Die Abmessungen des Katheters sowie die für die einzelnen Elemente eingesetzten Materialien können denen bei der Anordnung nach Fig. 1 entsprechen.

Ein guter Kontakt mit dem Epikard und damit die Gewinnung aussagekräftige Meßwerte mit der Anordnung bei gleichzeitig guter Einsetzbarkeit werden durch eine semiflexible oder gesteuert flexible Ausbildung der Schenkel 103a und 103b begünstigt. Eine gesteuerte Flexibilität kann - was in den Figuren nicht dargestellt ist - auf einfache Weise über eine Ausbildung der Elektrodenträger als gas- oder flüssigkeitsgefüllte Teile und ihre Verbindung mit einem Drucksteuerventil, als Elemente mit temperaturabhängiger Steifigkeit und einer integrierten Heiz- oder Kühlvorrichtung (etwa einem Peltierelement) oder auf ähnliche Weise realisiert werden.

Die Erfindung beschränkt sich in ihrer Ausführung nicht auf das vorstehend angegebene bevorzugte Ausführungsbeispiel. Vielmehr ist eine Anzahl von Varianten denkbar, welche in dem durch die Ansprüche abgesteckten Rahmen von der dargestellten Lösung auch bei anders gearteten Ausführungen Gebrauch machen.

Dabei ist der Einsatz auch nicht auf perikardiale Messungen oder Stimulationen beschränkt, sondern gegenüber den Beispielen in den Abmessungen und der Geometrie entsprechend modifizierte Anordnungen sind auch endokardial einsetzbar.

## Patentansprüche

1. Endoskopisch einsetzbare Elektrodenanordnung (1; 101), insbesondere für die epikardiale Tachykardiekartierung und -behandlung, mit einer flexiblen Hülse (2b; 102b) mit elektrischen Zuleitungen (21; 121) und einem Betätigungselement (4; 104), einem sich distal an die flexible Hülse anschließenden, im wesentlichen zylindrischen Katheterkopf (2a; 102a) und einem zum Einführen in den Körper im Katheterkopf aufgenommenen und mit Hilfe des Betätigungselements in seiner Lage und/oder geometrischen Gestalt veränderbaren Elektrodenträger (31; 103a, 103b),
**dadurch gekennzeichnet**, daß
der Elektrodenträger ein aus dem Katheterkörper (2; 102) ausklapp- oder auffaltbares flächiges Gebilde aufweist, das im ausgeklappten bzw. aufgefalteten Zustand in mindestens einer die Katheterlängsachse enthaltenden Ebene im wesentlichen V- oder U-Gestalt annimmt.

2. Elektrodenanordnung nach Anspruch 1 , **dadurch gekennzeichnet**, daß der Elektrodenträger mindestens zwei langgestreckte, gegeneinander verschwenkbare Schenkel (103a, 103b) aus mindestens während des Einführens steifem oder halbsteifem Material aufweist, deren Längsachsen zum Einführen in den Körper im wesentlichen parallel zur Katheterlängsachse liegen und im ausgeklappten bzw. aufgefalteten Zustand mit dieser einen spitzen Winkel einschließen.

3. Elektrodenanordnung nach Anspruch 1 oder 2, **dadurch gekennzeichnet**, daß der Elektrodenträger (31) eine gitter- bzw. netzartige Struktur aus flexiblem Material mit halbsteifen. elastischen Aufspreizungselementen (32-35) aufweist.

4. Elektrodenanordnung nach Anspruch 1 oder 2, **dadurch gekennzeichnet**, daß der Elektrodenträger eine vollflächige Lage aus flexiblem Material mit halbsteifen, elastischen Aufspreizungselementen aufweist.

5. Elektrodenanordnung nach einem der vorangehenden Ansprüche, **dadurch gekennzeichnet**, daß auf dem Elektrodenträger (31; 131) eine Mehrzahl einzelner, voneinander isolierter Elektroden (37; 137) angeordnet ist.

6. Elektrodenanordnung nach Anspruch 5, **dadurch gekennzeichnet**, daß die Anzahl der einzelnen Elektroden (37; 137) zwischen 10 und 200 liegt.

7. Elektrodenanordnung nach Anspruch 5 oder 6, da**durch gekennzeichnet**, daß der Abstand der einzelnen Elektroden (37; 137) voneinander etwa 1 cm beträgt.

8. Elektrodenanordnung nach einem der Ansprüche 5 bis 7, **dadurch gekennzeichnet**, daß die Elektroden (37; 137) aus einem hoch leitfähigen biokompatiblen Material, insbesondere TiN oder IrO₂, bestehen.

9. Elektrodenanordnung nach einem der Ansprüche 5 bis 8, **dadurch gekennzeichnet**, daß die Elektroden (37; 137) im wesentlichen matrixartig angeordnet sind.

10. Elektrodenanordnung nach Anspruch 9 , **dadurch gekennzeichnet**, daß jede Spalte und Zeile der matrixartigen Anordnung mit einer separaten Zuleitung zum Abgreifen oder Zuführen einer elektrischen Spannung verbunden ist.

11. Elektrodenanordnung nach Anspruch 3 oder 4, **dadurch gekennzeichnet**, daß das flexible Material eine Widerstandsfolie aufweist und an seiner Umfangslinie eine Mehrzahl von Anschlüssen vorgesehen ist, von denen jeder mit einer separaten Zuleitung verbunden ist.

12. Elektrodenanordnung nach einem der vorangehenden Ansprüche, **dadurch gekennzeichnet**, daß die Länge des Katheterkopfes (2a; 102a) zwischen 10 und 15 cm und sein Durchmesser zwischen 1 und 3 cm liegt.

13. Elektrodenanordnung nach einem der vorangehenden Ansprüche, **dadurch gekennzeichnet**, daß der Katheterkopf (2a; 102a) und/oder der Elektrodenträger (31; 131) ein PTFE-, Polyamid- oder Polyethylensubstrat aufweisen.

14. Elektrodenanordnung nach einem der vorangehenden Ansprüche, **dadurch gekennzeichnet**, daß die Öffnungsweite des V- oder U-förmig aufgeklappten bzw. aufgefalteten Elektrodenträgers (31; 131) 10 bis 15 cm beträgt.

15. Elektrodenanordnung nach einem der Ansprüche 2 bis 14, **dadurch gekennzeichnet**, daß die Schenkel und/oder Aufspreizungselemente Mittel zum Steuern ihrer Steifigkeit derart aufweisen, daß sie beim Einführen in den Körper hohe und in Arbeitsstellung in Kontakt mit dem Herzen geringe Steifigkeit haben.

16. Elektrodenanordnung nach Anspruch 15, **dadurch gekennzeichnet**, daß die Mittel zum Steuern der Steifigkeit einen gas- oder flüssigkeitsgefüllten Hohlraum und ein Gas- oder Flüssigkeitsdrucksteuerventil umfassen.

17. Elektrodenanordnung nach Anspruch 15, **dadurch gekennzeichnet**, daß die Mittel zum Steuern der Steifigkeit tragende Elemente mit temparaturabhängiger Steifigkeit und eine Vorrichtung zum Verändern der Temperatur dieser Elemente umfassen.

18. Elektrodenanordnung nach einem der vorangehenden Ansprüche, **dadurch gekennzeichnet**, daß im Katheterkopf eine optische bzw. optoelektronische Anordnung zur visuellen oder videotechnischen Beobachtung des Untersuchungsortes vorgesehen ist und die Hülse eine entsprechende, mit der optischen Anordnung verbundene Signalleitung aufweist.

## Claims

1. An endoscopically insertable electrode arrangement (1,101), in particular for epicardial tachycardiac mapping and treatment, having a flexible sheath (2b,102b) with electrical conduits (21,121) and an actuating element (4,104), an essentially cylindrical catheter head (2a,102a) distally joining up with the flexible sheath and an electrode carrier (31,103a,103b) for introduction into the body accommodated in the catheter head and which is variable in its position and/or geometric shape
**characterised in that**
the electrode carrier has a flat structure which can be opened-out or unfolded from the catheter body (2,102), which in the opened-out or unfolded state, assumes essentially a V- or U-shape in at least one of the planes contained in the catheter longitudinal axis.

2. An electrode arrangement according to claim 1, characterised in that the electrode carrier has at least two, longitudinally extending, mutually pivotable side-pieces (103a,103b) made from rigid or semi-rigid material during the insertion, the longitudinal axes of which for insertion into the body are located essentially in parallel to the catheter longitudinal axis, and which in the opened-out or unfolded state incorporate a pointed angle with same.

3. An electrode arrangement according to claims 1 or 2, characterised in that the electrode carrier (31) has a lattice or net-like structure made from flexible material having semi-rigid elastic expanding elements (32-35).

4. An electrode arrangement according to claims 1 or 2, characterised in that the electrode carrier has a holohedral layer made from flexible material, having semi-rigid, elastic expanding elements.

5. An electrode arrangement according to any of the above claims, characterised in that a plurality of individual electrodes (37,137), insulated from one another, are arranged on the electrode carrier (31,131).

6. An electrode arrangement according to claim 5, characterised in that the number of individual electrodes (37,137) is between 10 and 200.

7. An electrode arrangement according to claims 5 or 6, characterised in that the distance between the individual electrodes (37,137) amounts to about 1 cm.

8. An electrode arrangement according to claims 5-7, characterised in that the electrodes (37,137) are comprised of a high conductivity, bio-compatible material, in particular TiN or IrO₂.

9. An electrode arrangement according to any of claims 5-8, characterised in that the electrodes (37,137) are arranged essentially matrix-like.

10. An electrode arrangement according to claim 9, characterised in that each gap and line of the matrix-like arrangement is connected to a separate supply line for tapping or supplying an electric voltage.

11. An electrode arrangement according to claims 3 or 4, characterised in that the flexible material has a resistance sheet, and that a plurality of connections are provided around its circumferential line, each of which being connected to a separate supply line.

12. An electrode arrangement according to any of the above claims, characterised in that the length of the catheter head (2a,102a) is between 10 and 15 cm, and its diameter is between 1 and 3 cm.

13. An electrode arrangement according to any of the above claims, characterised in that the catheter head (2a,102a) and/or the electrode carrier (31,131) have a PTFE-, polyamide- or polyethylene substrate.

14. An electrode arrangement according to any of the above claims, characterised in that the opening width of the V- or U-shaped opened-out or unfolded electrode carrier (31,131) amounts to 10 to 15 cm.

15. An electrode arrangement according to any of claims 2-14, characterised in that the side pieces and/or the expanding elements have means for controlling their rigidity in such a way that on insertion into the body, they have high rigidity and in the working position in contact with the heart, they have low rigidity.

16. An electrode arrangement according to claim 15, characterised in that the means for controlling the rigidity encompasses a gas or fluid-filled cavity, and a gas or fluid pressure control valve.

17. An electrode arrangement according to claim 15, characterised in that the means for controlling the rigidity encompasses bearing elements having temperature-dependant rigidity and a device for varying the temperature of these elements.

18. An electrode arrangement according to any of the above claims, characterised in that an optic or opto-electronic arrangement for visual or video-technical observation of the investigation is provided in the catheter head, and the sheath has a corresponding signal line connected to the optic arrangement.

## Revendications

1. Agencement d'électrodes (1 ; 101) utilisable en endoscopie, en particulier pour la cartographie épicardique et le traitement de la tachycardie, comportant une gaine flexible (2b ; 102b) avec des conducteurs électriques (21 ; 121) et un élément d'actionnement (4 ; 104), une tête de cathéter (2a ; 102a) sensiblement cylindrique, qui se raccorde en position distale à la gaine flexible, et un porte-électrodes (31 ; 103a, 103b) contenu dans la tête de cathéter pour l'insertion dans le corps et dont la position et/ou la configuration géométrique sont modifiables au moyen de l'élément d'actionnement, caractérisé en ce que le porte-électrodes comporte une configuration en nappe pouvant se déployer ou s'épanouir depuis la tête de cathéter (2 ; 102) qui revêt, à l'état déployé ou épanoui, une configuration sensiblement en V ou en U dans au moins un plan contenant l'axe longitudinal du cathéter.

2. Agencement d'électrodes selon la revendication 1, caractérisé en ce que le porte-électrodes comporte au moins deux branches (103a, 103b) allongées, pouvant pivoter l'une par rapport à l'autre en un matériau rigide ou semi-rigide au moins pendant l'insertion, dont les axes longitudinaux sont situés sensiblement parallèlement à l'axe longitudinal du cathéter pour l'insertion dans le corps et forment un angle aigu avec celui-ci à l'état déployé ou épanoui.

3. Agencement d'électrodes selon la revendication 1 ou 2, caractérisé en ce que le porte-électrodes (31) comporte une structure de type grille ou réseau en matériau flexible avec des éléments d'épanouissement (32-35) élastiques semi-rigides.

4. Agencement d'électrodes selon la revendication 1 ou 2, caractérisé en ce que le porte-électrodes comporte une couche sur toute la surface en un matériau flexible avec des éléments d'épanouissement élastiques semi-rigides.

5. Agencement d'électrodes selon l'une des revendications précédentes, caractérisé en ce qu'une multiplicité d'électrodes individuelles (37 ; 137) isolées les unes par rapport aux autres sont disposées sur le porte-électrodes (31 ; 131).

6. Agencement d'électrodes selon la revendication 5, caractérisé en ce que le nombre des électrodes individuelles (37 ; 137) est situé entre 10 et 200.

7. Agencement d'électrodes selon la revendication 5 ou 6, caractérisé en ce que la distance entre les électrodes individuelles (37 ; 137) est d'environ 1 cm.

8. Agencement d'électrodes selon l'une des revendications 5 à 7, caractérisé en ce que les électrodes (37 ; 137) consistent en un matériau biocompatible hautement conducteur, en particulier en TiN ou en IrO₂.

9. Agencement d'électrodes selon l'une des revendications 5 à 8, caractérisé en ce que les électrodes (37 ; 137) sont agencées sensiblement selon une matrice.

10. Agencement d'électrodes selon la revendication 9, caractérisé en ce que chaque colonne et chaque ligne de l'agencement selon une matrice sont reliées à un conducteur séparé pour prélever ou transmettre une tension électrique.

11. Agencement d'électrodes selon la revendication 3 ou 4, caractérisé en ce que le matériau flexible comporte une feuille de résistance et en ce qu'il est prévu sur sa ligne périphérique une multiplicité de connexions reliées chacune à un conducteur séparé.

12. Agencement d'électrodes selon l'une des revendications précédentes, caractérisé en ce que la longueur de la tête de cathéter (2a ; 102a) est située entre 10 et 15 cm et son diamètre est situé entre 1 et 3 cm.

13. Agencement d'électrodes selon l'une des revendications précédentes, caractérisé en ce que la tête de cathéter (2a ; 102a) et/ou le porte-électrodes (31 ; 131) comportent un substrat en PTFE, en polyamide ou en polyéthylène.

14. Agencement d'électrodes selon l'une des revendications précédentes, caractérisé en ce que l'ampleur d'ouverture du porte-électrodes (31 ; 131) déployé ou épanoui en forme de V ou U est de 10 à 15 cm.

15. Agencement d'électrodes selon l'une des revendications 2 à 14, caractérisé en ce que les branches et/ou les éléments d'épanouissement comportent des moyens pour commander leur rigidité de telle manière qu'ils ont une rigidité élevée lors de l'insertion dans le corps et une faible rigidité en position de travail en contact avec le coeur.

16. Agencement d'électrodes selon la revendication 15, caractérisé en ce que les moyens pour commander la rigidité comprennent une cavité remplie de gaz ou de liquide et une soupape de commande de pression de gaz ou de liquide.

17. Agencement d'électrodes selon la revendication 15, caractérisé en ce que les moyens pour commander la rigidité comprennent des éléments porteurs à rigidité dépendante de la température et un dispositif pour modifier la température de ces éléments.

18. Agencement d'électrodes selon l'une des revendications précédentes, caractérisé en ce qu'il est prévu dans la tête de cathéter un agencement optique ou optoélectronique pour l'observation visuelle ou par la technique vidéo du site d'examen et la gaine comporte un conducteur de signaux correspondant, relié à l'agencement optique.
